# EUROPEAN PATENT SPECIFICATION

(11) **EP 4 472 592 B1**
(45) Date of publication and mention of the grant of the patent: **30.09.2026**
(21) Application number: 23705051.3
(22) Date of filing: 06.02.2023
(51) Int. Cl.: A61F 11/08

(54) **ADJUSTABLE ACOUSTO-MECHANICAL ELEMENT FOR A HEARING PROTECTION DEVICE**
EINSTELLBARES AKUSTISCH-MECHANISCHES ELEMENT FÜR EINE GEHÖRSCHUTZVORRICHTUNG
ÉLÉMENT ACOUSTO-MÉCANIQUE RÉGLABLE POUR UN DISPOSITIF DE PROTECTION AUDITIVE

(30) Priority: 04.02.2022 GB 202201473
(43) Date of publication of application: 11.12.2024
(73) Proprietor: SINTEF TTO AS, 7034 Trondheim (NO)
(72) Inventor: GJESSING, Jo, 0314 Oslo (NO); ANGELSKÅR, Hallvard, 0314 Oslo (NO); KVALØY, Olav, 0153 Oslo (NO)
(74) Representative: Dehns
(86) International application number: PCT/GB2023/050259
(87) International publication number: WO 2023/148507

(56) References cited:
- EP-A2- 2 124 477
- WO-A1-2017/203218
- WO-A1-2019/147131

## Description

The present invention relates to hearing protection used, for example, to reduce the intensity of sounds experienced by a user. Particularly it relates to an adjustable acousto-mechanical element for a hearing protection device.

Exposure to high intensity noises can cause damage to a person's hearing. The damaging effects are increased when a person is frequently exposed to loud noises. In extreme cases, frequent exposure to loud noises can cause noiseinduced hearing loss. Therefore, in order to protect hearing it is necessary to reduce the effects of continuous, intermittent and impact noises. As a result of the increasing awareness of the damaging effects of loud noises, for example from industrial sources, there are now various industry requirements for personnel to use ear protection. There are many situations in which personnel may be exposed to loud noises, for example when operating loud machinery. A common form of noise protection widely used are earplugs, these reduce the intensity of the sound entering a person's ears and thus reduce the damaging effects of high intensity noises.

There are two main types of earplugs that are commonly used: passive earplugs and active earplugs. Passive earplugs attenuate the intensity of all levels of sound equally, i.e. they provide a uniform level of attenuation, regardless of the intensity of sound present, for example, a reduction of 20 dB. Passive earplugs come in various forms including: foam, silicon, flanged and custom moulded earplugs. Passive earplugs are typically inserted into a user's ear canal. Passive earplugs use the material of the earplug itself to attenuate the sound which passes through it. As the incident sound passes through the earplug, the sound is attenuated by the material of the earplug. Some sound will propagate through the earplug and pass out of the earplug into the air volume of the user's ear canal where it will be detected by the user. The intensity of the sound will be reduced and thus the risk of damage to the user's hearing may be reduced. Unfortunately, however, such passive earplugs also change other properties of the sound as the attenuation is usually frequency dependent and as a result the sound quality is often reduced. This may be problematic, for example for musicians as they need to hear sounds in high fidelity to ensure that what they hear is actually what is being played.

In addition, if they are inserted properly, the fixed level of attenuation provided by passive earplugs is relatively high. As a result, users of passive earplugs typically have to periodically remove them in order to be communicate orally with fellow workers. The inconvenience associated with having to repeatedly remove and replace the earplugs, depending on the noise levels, may lead to reduced compliance with requirements to wear the earplugs in certain situations.

The aforementioned disadvantages can be overcome using active earplugs which contain circuitry that detects the incident sound. Systems will typically also contain a transducer projecting sound into the ear canal, at a controlled level. Active earplugs can employ control circuitry to apply different levels of attenuation at different times or different frequencies - known as 'adaptive attenuation'. One of the disadvantages of active earplugs is that they are often relatively expensive due to their complex electrical components such as speakers and microphones. Additionally, active earplugs often have a relatively high power consumption due to the need to constantly monitor and replay detected sound.

WO 2017/203218 A1 discloses a device for insertion into an ear canal of a mammalian subject which defines at least one sound path therethrough. The sound path has an attenuating arrangement therein which has a first configuration having a first level of attenuation and a second configuration having a second level of attenuation, higher than the first level.

WO 2019/147131 A1 discloses an acoustic filter.

EP 2124477 A2 discloses an earphone device and sound generating apparatus. The present invention seeks to address or mitigate the problems outlined above and according to a first aspect there is provided an adjustable acousto-mechanical element for a hearing protection device according to claim 1.

The applicant has recognised that with hearing protection devices in accordance with the invention, the level of acoustic loss introduced can be controlled by varying the dimensions of the sound path formed by the opening in the first portion. This control over the acoustic loss means that the amount of hearing protection provided by the acousto-mechanical element can be tailored to the current sound conditions. For example, when the level of ambient noise is very high, a high level of acoustic loss is needed, but when the level of ambient noise is lower, the level of acoustic loss provided by the acousto-mechanical element can be reduced so as to not interfere with a user's awareness of their surroundings.

Under the electrical circuit analogy for analysis of acoustic systems which will be familiar to those skilled in the art, acoustic loss is equivalent to a resistance and has an inverse cube relationship to the width of the opening. Since the attenuation is provided in the form of acoustic loss, it opens up the possibility of designing the hearing protection device to provide a desired level of attenuation
By controlling the acoustic loss using an adjustable opening between two portions, hearing protection devices in accordance with the present invention can be constructed more simply than prior solutions which involve complex electronic circuitry. This in turn leads to a smaller, and more cost effective hearing protection device.

The use of piezoelectric material means that the adjustable acousto-mechanical element can be more compact than an element with a moveable portion which is actuated mechanically. The adjustable acousto-mechanical element of the present invention may therefore provide the advantages associated with an adjustable acoustic loss element, whilst being compact enough to be more readily used in ear protection devices such as earplugs which are inserted into an ear canal.

In addition, the piezoelectric material can be designed to consume low power even when actuated thus keeping the power consumption of the device lower than in conventional circuitry.

In a set of embodiments, the second portion comprises a plurality of elongate fingers. These may be easier to move and control than a single element of equivalent surface area. Further, since the length of the slit between the 'finger' and the surrounding structure defines the acoustic loss, the finger structure can be optimized to increase this length and therefore increase the efficiency of the element in providing a desired acoustic loss.

In a set of embodiments, the second portion comprises a connecting band. The plurality of elongate fingers may each connect at their bases to the connecting band.

In a set of embodiments, the actuation portion comprises piezoelectric material which is provided on both the elongate fingers and the connecting band.

In a set of embodiments, the actuation portion comprises piezoelectric material which is provided on the connecting band only (i.e. no piezoelectric material is present on the elongate fingers). In such embodiments, the connecting band may be larger than in embodiments where piezoelectric material is provided on the fingers.

In a set of embodiments, the first and second portions are arranged such that, in a non-actuated state, a gap is present between the first and second portions, the gap forming at least part of a sound path. This may allow for a desired upper limit to the level of attenuation provided and may also avoid potential problems that might arise through contact between the first and second portions.

In a set of embodiments, the first and second portions are arranged such that at least part of the first portion overlaps at least part of the second portion.

In a set of embodiments, the first and second portions are offset, such that, in a non-actuated state, a tortuous path is formed between the overlapping parts of the first and second portions. The overlap which is created by the offset can be tailored to increase the range of acoustic losses provided throughout the actuation of the element.

In a set of embodiments, the first portion has a plurality of discrete openings, and the acousto-mechanical element comprises a respective plurality of second portions configured to at least partially close the plurality of openings.

According to a second aspect there is provided a hearing protection device according to claim 9.

In a set of embodiments, the hearing protection device includes a user operable input to adjust the first adjustable acousto-mechanical element. When a user hears, or expects to hear, a particular type of sound, they may operate the user operable to adjust the adjustment arrangement and thus alter the acoustic response of the at least one sound path.

In a set of embodiments the device further comprises a controller arranged to control the adjustment arrangement so as to alter the acoustic response of the at least one sound path. The inclusion of a controller may allow the device to control the adjustment arrangement in a more sophisticated manner in order to provide the most appropriate acoustic response of the sound path. It may, for example, try to achieve the highest sound quality whilst providing the necessary amount of attenuation. The controller may comprise a series of other components connected thereto or integrally provided therewith, for example a microphone or a transceiver. Such a set of embodiments may also advantageously automatically adjust the acoustic response of the sound path based on a detected sound environment in which the device is present. Additionally, or alternatively, the device may comprise a user input, e.g. in the form of at least one button, which enables the user to select operation of the device. This may, for example, allow a user to select a particular mode of operation.

The first acousto-mechanical element could be used alone. In a set of embodiments however it comprises part of a set of acoustic elements, e.g. including an acoustic filter, controlling the frequency response of the attenuation.

In a set of embodiments, the hearing protection device further comprises a second adjustable acousto-mechanical element, arranged acoustically in series with the first adjustable acousto-mechanical element, comprising an adjustable membrane.

As explained in more detail in International Patent application PCT/GB2020/050755, which is published as WO 2020/188296, the membrane may provide an acoustic capacitance.

Following the aforementioned electrical analogy, when the membrane is tensioned the acoustic capacitance will decrease.

As this capacitance is in series with the acoustic capacity of the ear canal, but also the resistance and inductance of the transmission path through the gap between the first and second portions of the first acousto-mechanical element, this will cause the attenuation to increase, but will also change the frequency response. The acoustic resistance, inductance and capacitance can be tuned so that the frequency response matches the natural frequency response of the human ear so that the frequency spectrum perceived by a user is essentially flat.

In a set of embodiments the hearing protection device comprises an adjustment arrangement for simultaneously adjusting the first and second adjustable acousto-mechanical elements to alter an acoustic response of the at least one sound path.

The Applicant has recognised that with the above arrangement, it is possible to achieve an acoustic response of the sound path which does not significantly reduce the quality of the sound passing through the sound path whilst maintaining the ability to control the sound, e.g. by attenuating the sound. This is because it allows the changes in the first acousto-mechanical element and the membrane as they are adjusted to complement one another in maintaining a favourable acoustic response.

As will be understood by those skilled in the art, the acoustic response of the sound path should be understood to be how the sound path affects the sound which passes through it. The acoustic response of the sound path may change the frequency, amplitude and/or phase of the sound passing through it and thus ultimately change the sound heard by a user of the device.

The adjustment arrangement may comprise any suitable arrangement for adjusting the first adjustable acousto-mechanical element and the second adjustable acousto-mechanical portion. In a set of embodiments the adjustment arrangement comprises a second actuator for adjusting the second adjustable acousto-mechanical element. The first and second actuators may, for example, be connected to a single controller capable of simultaneously controlling each of the first and second adjustable acousto-mechanical elements.

In a set of embodiments, the adjustable acousto-mechanical element of the first aspect, and/or the first adjustable acousto-mechanical element of the hearing protection device of the second aspect may be configured to operate as a microphone.

In such embodiments, the moveable portion may be configured to vibrate in response to sound passing through the opening such that this vibration deforms the piezoelectric material, creating a variation of the voltage across the piezoelectric material. The hearing protection device can thus monitor the voltage to determine properties (such as amplitude and frequency) of the sound passing through the acousto-mechanical element.

In a set of embodiments, the hearing protection device is configured to use the determined properties of the sound passing through the first acousto-mechanical element to decide how much loss should be introduced by the first acousto-mechanical element.

Embodiments of the invention will now be described, by way of example only, with reference to the accompanying drawings in which:
Fig. 1 is a plan view of an element in accordance with an embodiment of the present invention
Fig. 2 is a plan view showing the piezo-electric actuation portion of an element in accordance with an embodiment of the present invention;
Fig. 3 is a profile view of an element in accordance with an embodiment of the present invention in a non-actuated state;
Fig. 4 is a profile view of the element of Fig. 3, in an actuated state;
Fig. 5 is a profile view of an element in accordance with a further embodiment of the present invention in a non-actuated state;
Fig. 6 is a profile view of the element of Fig. 5 in an actuated state;
Fig. 7 is a plan view of an element in accordance with a further embodiment of the invention;
Fig. 8 is a plan view showing a moveable portion of an element in accordance with a further embodiment of the present invention;
Fig. 9 is a schematic diagram of a hearing protection device according to an embodiment of the invention.

### DETAILED DESCRIPTION

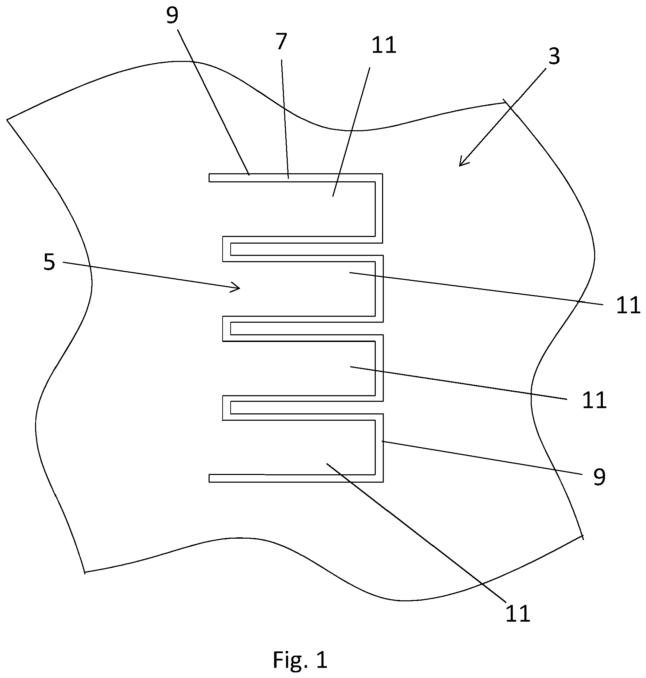

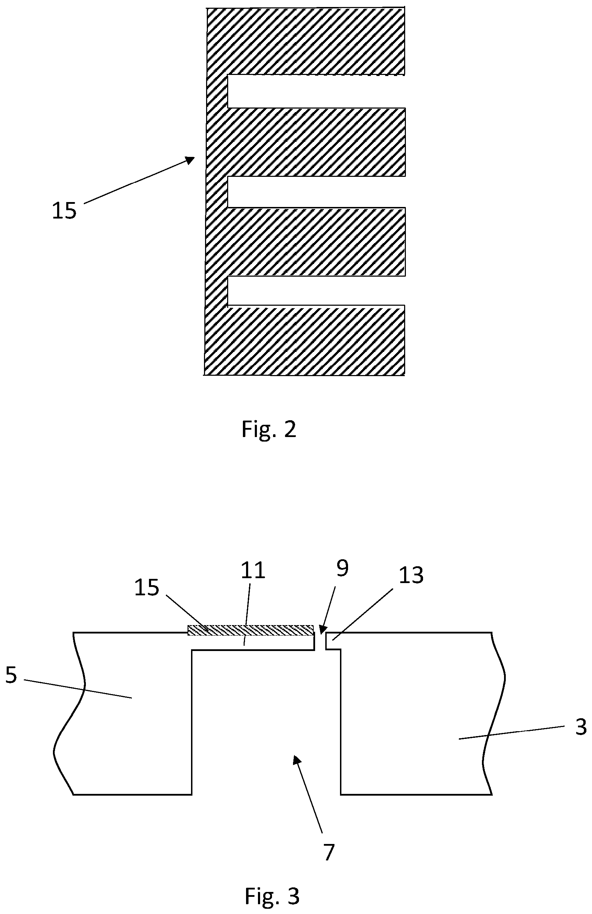

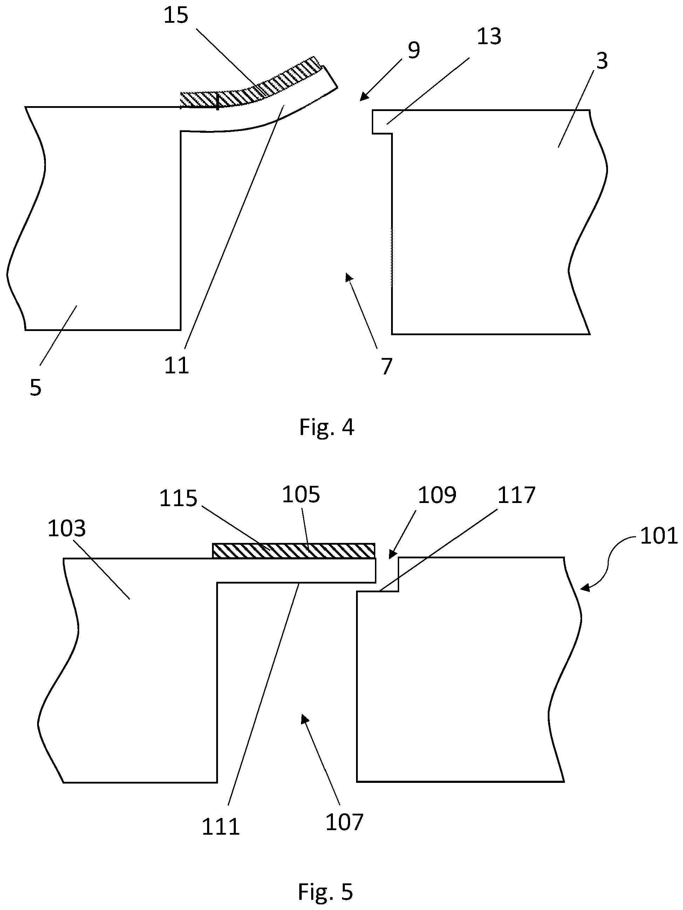

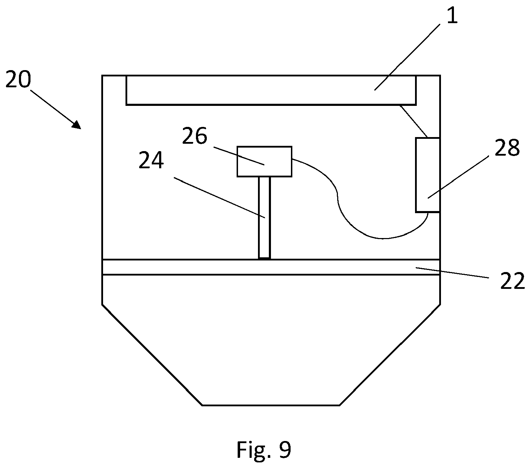

Fig. 1 is a schematic diagram of an acoustic attenuating element 1 which can be used in a hearing protection device as described below with reference to Fig. 9. The attenuating element 1 includes a first, fixed portion 3 and a second, moveable portion 5 which are both formed from the same piece of silicon. An opening 9 is formed in the fixed portion 3, and partially closed by the fixed portion 3 such that a narrow gap remains around part of the perimeter of the moveable portion 5. The opening 9 provides an adjustable acoustic path through the element as will be explained further below. A portion of the perimeter of the moveable portion 5 is joined to the fixed portion such that a flexible hinge is formed between the fixed portion 3 and the moveable portion 5. It will be understood that the curved outline of the fixed portion 3 is intended to illustrate that only part of the fixed portion 3 is shown. The moveable portion 5 has a plurality of fingers 11. As shown in Figs. 3 and 4, the moveable portion 5 has a sheet of piezoelectric material 15 disposed on its surface. The piezoelectric material 15 is omitted from Fig. 1 for clarity.

In the illustrated embodiment, the fixed portion 3 and the moveable portion 5 are formed of silicon, although it will be understood that any suitable material may be used.

The actuating piezoelectric material layer 15 is shown in isolation in Fig. 2. As may be inferred from the shape of this, in the illustrated embodiment, the piezoelectric material 15 is deposited both on the fingers 11, and on a connecting band of the moveable portion 5 to the left of the fingers. In other embodiments, the moveable portion may only comprise the fingers 11 such that the piezoelectric material 15 may be deposited only on the fingers 11.

Fig. 3 is a cross-section of the element 1. It will be understood that in Figures 3 to 6, the curved lines defining the fixed portion are intended to illustrate that the fixed portion 3 is larger than the part shown and that in embodiments having a plurality of moveable portions 5, the plurality of moveable portions may be provided within a single fixed portion 3.

The gap 9 between the end of a finger 11 and the fixed portion 3 can be seen. At the edge of the fixed portion 3 there is a lip 13 which extends towards the corresponding finger 11. Disposed on the top surface of the moveable portion 5 is the layer of piezoelectric material 15 shown in Fig. 2. It can also be seen that the silicon is etched away beneath the finger 11 to form a cavity 7. This enables the finger part of the moveable portion 5 to deflect more substantially as seen in Fig. 4.

The operation of the attenuating element 1 will now be described with reference to Fig. 3 and Fig. 4. Fig. 3 shows the element 1 in a non-actuated state. In this state, the finger 11 is in its resting position such that the acoustic path through the element is determined by the length and width of the gap 9 between the lip 13 of the fixed portion 3, and the finger 11 of the moveable portion 5. As a voltage is applied to the piezoelectric material 15, the piezoelectric material 15 contracts. The reduced thickness of the silicon that defines the fingers 11 makes them sufficiently flexible to be deformed by the contraction of the piezoelectric material 15. As such, upon application of a voltage to the piezoelectric material 15, the fingers 11 of the moveable portion 5 are made to move up, away from the fixed portion 3 which increases the effective width of the gap 9 between the edge of the moveable portion 5, and the lip 13. The dimensions of the sound path through the cavity 7 and the gap 9 are therefore increased. Although Fig. 3 and Fig. 4 are shown in profile, it will be understood that as the fingers 11 are deflected away from the fixed portion 3, the width of the gap 9 on all three sides of the finger will increase.

Adjusting the width of the gap 9 may serve to adjust the specific acoustic properties of the sound path. For example, decreasing the width of the gap 9 will typically increase the effective acoustic mass and the acoustic loss of the gap and vice versa. The terms acoustic mass and acoustic loss are well known to those skilled in the art.

By applying different levels of voltage to the piezoelectric material 15, the exact dimensions of the gap 9 can be adjusted, and hence the level of attenuation can be precisely altered.

It will be understood that although the moveable portion 5 of the illustrated embodiments are arranged to move away from the fixed portion 3 when actuated, the element 1 may instead be configured such that the moveable portion is at greater separation from the fixed portion 5 in a resting position, and made to move towards the fixed portion 3 when actuated.

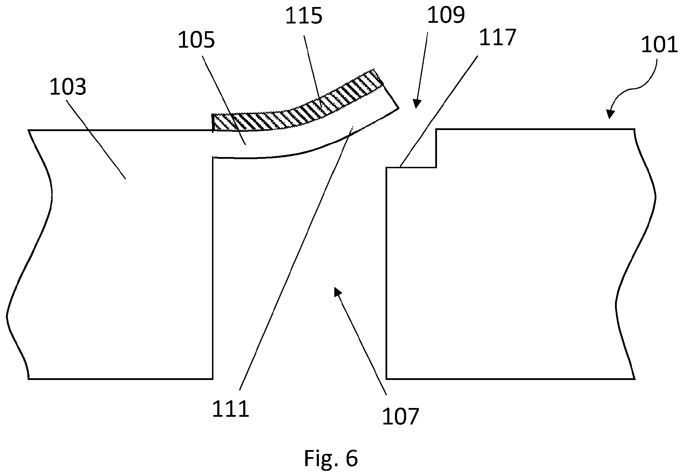

Figs. 5 and 6 show a cross-section view of an element 101 according to a second embodiment of the invention in actuated and non-actuated states respectively. In the second embodiment, the fixed portion 103 has a lowered shelf 117 around the periphery of the gap 109, and each edge of the fingers 111 is vertically offset from but overlaps part of the lowered shelf 117 such that the gap 109 forms a tortuous path between the fixed portion 103 and fingers 111 of the moveable portion 105. The arrangement of the second embodiment may lead to a different variation of the acoustic properties upon deflection of the fingers 111. In this way the variation of properties may be tailored to the needs of a specific application.

In the illustrated embodiments, the moveable portion 5, 105 is integrally formed with the fixed portion 3, 103 but it will be understood that these portions may be separately formed. Further, in both the first and second embodiments, the moveable portion 5, 105 is configured to bend in response to the deformation of the piezoelectric material 15, 115 provided on its surface. It will however be understood that the movement of the moveable portion 5, 105 may be realised in different ways. For example, the moveable portion 5, 105 may be rigid, and may instead be formed separately to the fixed portion 3, 103, and connected to the fixed portion via a hinge such that the moveable portion 5, 105 may move relative to the fixed portion 3, 103.

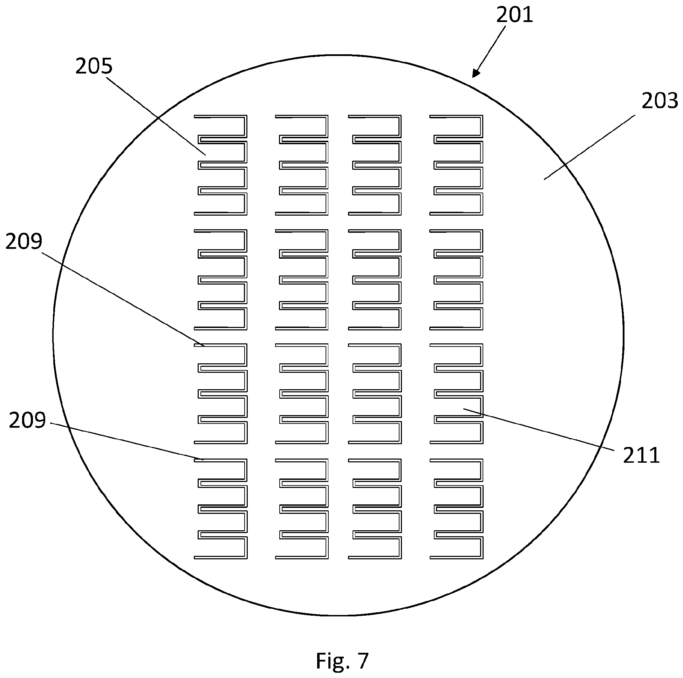

Fig. 7 shows an element 201 according to a further embodiment, with a fixed first portion 203 which defines a plurality of discrete gaps 209 and thus respective moveable portions 205, each with a plurality of fingers 211. In Fig. 7, the fixed first portion 203 and the plurality of moveable portions 205 are similar to those of the first embodiment shown in Fig. 3 and Fig. 4, but it will be understood than an arrangement such as that shown in Fig. 7 is equally compatible with a fixed portion 203 and plurality of moveable portions 205 according to the second embodiment shown in Fig. 6 and Fig. 7. When a plurality of moveable portions 205 are present, the moveable portions 205 may be actuated simultaneously, or they may be independently actuated to provide more precise control over the acoustic loss provided by the element 201 as a whole. In Fig. 7, the fixed portion 203 is circular, but it will be understood that the fixed portion 203 may be any suitable shape.

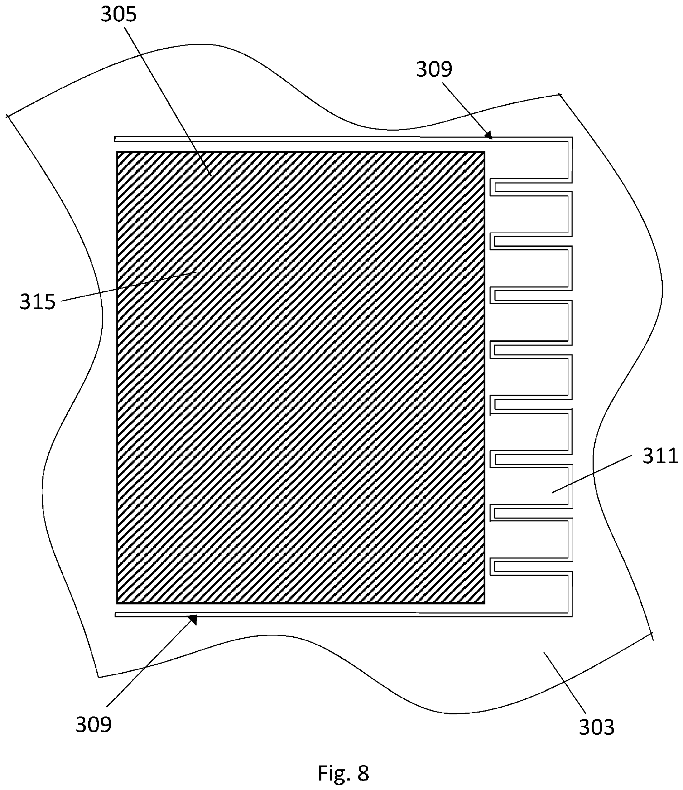

Fig. 8 shows a plan view of a moveable portion 305 in accordance with an alternative embodiment. In the moveable portion 305 of Fig. 8, the piezoelectric material 315 is only provided on the common connecting band part of the moveable portion 305, but the fingers 311 interact with a corresponding fixed portion 303 to alter the dimensions of the gap 309 and hence adjust the acoustic response as explained in relation to earlier embodiments. In this embodiment the cavity (not shown) beneath the moveable portion may be extended to allow greater movement of the moveable element 205 as a whole.

Fig. 9 schematically shows a hearing protection device 20 which includes an adjustable acousto-mechanical element 1, the structure and operation of which has been discussed above, and a second adjustable acousto-mechanical element, in the form of an adjustable membrane 22. The adjustable membrane provides an acoustic capacitance which can be varied by applying a variable tension to it by means of an adjustable member 24 operated by an actuator 26. The way in which a variable tension may be applied by the adjustable member is described in further detail in WO2021/130482. The actuator 26 operating the adjustable member 24 is operated by a controller 28 which also applies a voltage to the piezoelectric material 15. The first adjustable acousto-mechanical element 1, and the second adjustable acousto-mechanical element 22 are arranged acoustically in series and adjusted simultaneously by the controller 28. As described in WO2020/188296 and WO2021/130482, by providing the two acousto-mechanical elements in series, it is possible to achieve an acoustic response of the sound path which does not significantly reduce the quality of the sound passing through the sound path whilst maintaining the ability to control the sound, e.g. by attenuating the sound. This is because it allows the changes in the gap 9 and the membrane as they are adjusted to complement one another in maintaining a favourable acoustic response. The changes in the acoustic loss and acoustic mass introduced by the gap 9 and the membrane can be tuned such that the change in frequency response introduced by the hearing protection device matches the natural frequency response of the human ear so that the frequency spectrum perceived by a user is essentially flat.

As will be understood by those skilled in the art, the acoustic response of the sound path should be understood to be how the sound path affects the sound which passes through it. The acoustic response of the sound path may change the frequency, amplitude and/or phase of the sound passing through it and thus ultimately change the sound heard by a user of the device.

Although the adjustable acousto-mechanical element has been discussed in relation to its use as an element for introducing acoustic loss, the applicant has appreciated that the adjustable acousto-mechanical element may also simultaneously operate as a microphone. When sound passes through the gap 9, 109, 209, the fingers 11, 111, 211, will vibrate in response to that sound, and hence the piezoelectric material 15, 115 will be deformed slightly from its rest position set by the applied voltage (dependent on required loss). This deformation will create a perturbation in the voltage across the piezoelectric material, which is dependent on the amplitude and frequency of the deformation and hence the amplitude and frequency of the sound passing through the gap 9. 109. 209.

Embodiments of the invention implementing an adjustable acousto-mechanical element in a hearing protection device may therefore apply a voltage to the piezoelectric material to adjust the amount of loss introduced, whilst simultaneously measuring the variation in the voltage across the piezoelectric material 15, 115 to determine the properties of the sound passing through the gap 9, 109, 209 which may in turn be used as part of a feedback system to decide how much loss should be introduced by the acousto-mechanical element, i.e. how much hearing protection is needed. Such a microphone is likely to have a low sensitivity, but since hearing protection is only required when ambient sound levels are high, this low sensitivity does not preclude use of the microphone as part of a hearing protection device.

In each of the illustrated embodiments, the moveable portion 5 is actuated by applying a voltage to a layer of piezoelectric material provided on its surface. It will be understood that the moveable portion may be actuated in any suitable way.

## Claims

1. An adjustable acousto-mechanical element (1) for a hearing protection device, comprising:
a first portion (3) forming an opening (9) which forms at least part of a sound path;
a second portion (5) configured to at least partially close the opening (9); and
an actuation portion comprising piezoelectric material (15) configured to exert a force on the second portion (5),
wherein, the force exerted by the actuation portion (15) causes at least part of the second portion (5) to move towards/away from the first portion (3), varying the dimensions of the opening (9) to alter an acoustic response of the sound path,
**characterized in that**:
the piezoelectric material (15) of the actuation portion is provided on the second portion (5), and **in that**
the second portion (5) is configured to deform relative to the first portion (3) upon application of a voltage to the piezoelectric material (15).

2. The adjustable acousto-mechanical element (1) of claim 1, wherein the second portion (5) comprises a plurality of elongate fingers (11).

3. The adjustable acousto-mechanical element (1) of claim 2, wherein the second portion (5) comprises a connecting band.

4. The adjustable acousto-mechanical element (1) of claim 3, wherein the actuation portion comprises piezoelectric material (15) which is provided on both the elongate fingers (11) and the connecting band.

5. The adjustable acousto-mechanical element (1) of claim 3, wherein the actuation portion comprises piezoelectric material (15) which is provided on the connecting band only.

6. The adjustable acousto-mechanical element (1) of any preceding claim, wherein the first and second portions (3, 5) are arranged such that, in a non-actuated state, a gap (9) is present between the first and second portions (3, 5), the gap (9) forming at least part of a sound path.

7. The adjustable acousto-mechanical element (101) of any preceding claim, wherein the first and second portions (103, 105) are arranged such that at least part of the first portion (103) overlaps at least part of the second portion (105), optionally wherein the first and second portions (103, 105) are offset, such that, in a non-actuated state, a tortuous path is formed between the overlapping parts of the first and second portions (103, 105).

8. The adjustable acousto-mechanical element (201) of any preceding claim, wherein the first portion (203) has a plurality of discrete openings (209), and wherein the adjustable acousto-mechanical element (201) comprises a respective plurality of second portions (205) configured to at least partially close the plurality of openings (209).

9. A hearing protection device (20) for insertion into an ear canal of a mammalian subject, comprising a first adjustable acousto-mechanical element (1), wherein the first acousto-mechanical element (1) is an adjustable acousto-mechanical element (1) according to any preceding claim.

10. The hearing protection device (20) of claim 9, comprising a user operable input to adjust the first adjustable acousto-mechanical element (1).

11. The hearing protection device (20) of claim 9 or claim 10, comprising a controller (28) arranged to control the adjustment arrangement so as to alter the acoustic response of the at least one sound path.

12. The hearing protection device (20) of any of claims 9 to 11, comprising a user operable input, which enables the user to select operation of the device (20).

13. The hearing protection device (20) of any of claims 9 to 12, wherein the first acousto-mechanical element (1) comprises part of a set of acoustic elements, controlling the frequency response of the attenuation.

14. The hearing protection device (20) of any of claims 9 to 13, further comprising a second adjustable acousto-mechanical element (22), arranged acoustically in series with the first adjustable acousto-mechanical element (1), the second adjustable acousto-mechanical element (22) comprising an adjustable membrane.

15. The hearing protection device (20) of claim 14, comprising an adjustment arrangement for simultaneously adjusting the first and second adjustable acousto-mechanical elements (1, 22) to alter an acoustic response of the at least one sound path, optionally wherein the adjustment arrangement comprises a second actuator (26) for adjusting the second adjustable acousto-mechanical element (22).

## Patentansprüche

1. Einstellbares akustisch-mechanisches Element (1) für eine Gehörschutzvorrichtung, umfassend:
einen ersten Abschnitt (3), der eine Öffnung (9) bildet, die mindestens einen Teil eines Schallpfads bildet;
einen zweiten Abschnitt (5), der mindestens teilweise zum Schließen der Öffnung (9) ausgebildet ist; und
einen Betätigungsabschnitt, der piezoelektrisches Material (15) umfasst, das so ausgebildet ist, dass es eine Kraft auf den zweiten Abschnitt (5) ausübt,
wobei die von dem Betätigungsabschnitt (15) ausgeübte Kraft mindestens einen Teil des zweiten Abschnitts (5) zu/von dem ersten Abschnitt (3) hin-/wegbewegt, wobei die Abmessungen der Öffnung (9) variiert werden, um eine akustische Reaktion des Schallpfads zu verändern,
**dadurch gekennzeichnet, dass**:
das piezoelektrische Material (15) des Betätigungsabschnitts auf dem zweiten Abschnitt (5) bereitgestellt ist, und dass
der zweite Abschnitt (5) so ausgebildet ist, dass er sich relativ zum ersten Abschnitt (3) verformt, wenn eine Spannung auf das piezoelektrische Material (15) angelegt wird.

2. Einstellbares akustisch-mechanisches Element (1) nach Anspruch 1, wobei der zweite Abschnitt (5) eine Vielzahl von länglichen Fingern (11) umfasst.

3. Einstellbares akustisch-mechanisches Element (1) nach Anspruch 2, wobei der zweite Abschnitt (5) ein Verbindungsband umfasst.

4. Einstellbares akustisch-mechanisches Element (1) nach Anspruch 3, wobei der Betätigungsabschnitt piezoelektrisches Material (15) umfasst, das sowohl an den länglichen Fingern (11) als auch an dem Verbindungsband bereitgestellt ist.

5. Einstellbares akustisch-mechanisches Element (1) nach Anspruch 3, wobei der Betätigungsabschnitt piezoelektrisches Material (15) umfasst, das nur am Verbindungsband bereitgestellt ist.

6. Einstellbares akustisch-mechanisches Element (1) nach einem vorstehenden Anspruch, wobei die ersten und zweiten Abschnitte (3, 5) so angeordnet sind, dass in einem nicht betätigten Zustand ein Spalt (9) zwischen den ersten und zweiten Abschnitten (3, 5) vorhanden ist, wobei der Spalt (9) mindestens einen Teil eines Schallpfads bildet.

7. Einstellbares akustisch-mechanisches Element (101) nach einem vorstehenden Anspruch, wobei die ersten und zweiten Abschnitte (103, 105) so angeordnet sind, dass mindestens ein Teil des ersten Abschnitts (103) mindestens einen Teil des zweiten Abschnitts (105) überlappt, wobei die ersten und zweiten Abschnitte (103, 105) wahlweise versetzt sind, so, dass im unbetätigten Zustand zwischen den sich überlappenden Teilen des ersten und zweiten Abschnitts (103, 105) ein gewundener Weg gebildet wird.

8. Einstellbares akustisch-mechanisches Element (201) nach einem vorstehenden Anspruch, wobei der erste Abschnitt (203) eine Vielzahl von diskreten Öffnungen (209) aufweist, und wobei das einstellbare akustisch-mechanische Element (201) eine jeweilige Vielzahl von zweiten Abschnitten (205) umfasst, die zum mindestens teilweisen Schließen der Vielzahl von Öffnungen (209) ausgebildet sind.

9. Gehörschutzvorrichtung (20) zum Einführen in einen Gehörgang eines Säugetiersubjekts, umfassend ein erstes einstellbares akustisch-mechanisches Element (1), wobei das erste akustisch-mechanische Element (1) ein einstellbares akustisch-mechanisches Element (1) nach einem vorstehenden Anspruch ist.

10. Gehörschutzvorrichtung (20) nach Anspruch 9, umfassend einen vom Benutzer bedienbaren Eingang zum Einstellen des ersten einstellbaren akustisch-mechanischen Elements (1).

11. Gehörschutzvorrichtung (20) nach Anspruch 9 oder Anspruch 10, umfassend eine Steuerung (28), die so angeordnet ist, dass sie die Einstellanordnung steuert, um die akustische Reaktion des mindestens einen Schallpfads zu verändern.

12. Gehörschutzvorrichtung (20) nach einem der Ansprüche 9 bis 11, umfassend einen vom Benutzer bedienbaren Eingang, der es dem Benutzer ermöglicht, den Betrieb der Vorrichtung (20) auszuwählen.

13. Gehörschutzvorrichtung (20) nach einem der Ansprüche 9 bis 12, wobei das erste akustisch-mechanische Element (1) Teil eines Satzes von akustischen Elementen umfasst, das die Frequenzreaktion der Dämpfung steuert.

14. Gehörschutzvorrichtung (20) nach einem der Ansprüche 9 bis 13, weiter umfassend ein zweites einstellbares akustisch-mechanisches Element (22), das akustisch in Reihe mit dem ersten einstellbaren akustisch-mechanischen Element (1) angeordnet ist, wobei das zweite einstellbare akustisch-mechanische Element (22) eine einstellbare Membran umfasst.

15. Gehörschutzvorrichtung (20) nach Anspruch 14, umfassend eine Einstellanordnung zum gleichzeitigen Einstellen der ersten und zweiten einstellbaren akustisch-mechanischen Elemente (1, 22) zur Veränderung einer akustischen Reaktion des mindestens einen Schallpfads, wobei die Einstellanordnung wahlweise einen zweiten Aktor (26) zum Einstellen des zweiten einstellbaren akustisch-mechanischen Elements (22) umfasst.

## Revendications

1. Élément acousto-mécanique (1) réglable pour un dispositif de protection auditive, comprenant :
une première portion (3) formant une ouverture (9) qui forme au moins une partie d'un trajet sonore ;
une deuxième portion (5) configurée pour fermer au moins partiellement l'ouverture (9) ; et
une portion d'actionnement comprenant un matériau piézoélectrique (15) configuré pour exercer une force sur la deuxième portion (5),
dans lequel la force exercée par la portion d'actionnement (15) amène au moins une partie de la deuxième portion (5) à se déplacer vers/à l'écart de la première portion (3), en faisant varier les dimensions de l'ouverture (9) pour modifier une réponse acoustique du trajet sonore,
**caractérisé en ce que** :
le matériau piézoélectrique (15) de la portion d'actionnement est prévu sur la deuxième portion (5), et **en ce que**
la deuxième portion (5) est configurée pour se déformer par rapport à la première portion (3) lors de l'application d'une tension au matériau piézoélectrique (15).

2. Élément acousto-mécanique (1) réglable selon la revendication 1, dans lequel la deuxième portion (5) comprend une pluralité de doigts allongés (11).

3. Élément acousto-mécanique (1) réglable selon la revendication 2, dans lequel la deuxième portion (5) comprend une bande de liaison.

4. Élément acousto-mécanique (1) réglable selon la revendication 3, dans lequel la portion d'actionnement comprend un matériau piézoélectrique (15) qui est prévu à la fois sur les doigts allongés (11) et la bande de liaison.

5. Élément acousto-mécanique (1) réglable selon la revendication 3, dans lequel la portion d'actionnement comprend un matériau piézoélectrique (15) qui est prévu uniquement sur la bande de liaison.

6. Élément acousto-mécanique (1) réglable selon une quelconque revendication précédente, dans lequel les première et deuxième portions (3, 5) sont agencées de sorte que, dans un état non actionné, un écart (9) est présent entre les première et deuxième portions (3, 5), l'écart (9) formant au moins une partie d'un trajet sonore.

7. Élément acousto-mécanique réglable (101) selon une quelconque revendication précédente, dans lequel les première et deuxième portions (103, 105) sont agencées de sorte qu'au moins une partie de la première portion (103) chevauche au moins une partie de la deuxième portion (105), facultativement dans lequel les première et deuxième portions (103, 105) sont décalées, de sorte que, dans un état non actionné, un trajet tortueux est formé entre les parties se chevauchant des première et deuxième portions (103, 105).

8. Élément acousto-mécanique réglable (201) selon une quelconque revendication précédente, dans lequel la première portion (203) présente une pluralité d'ouvertures (209) discrètes, et dans lequel l'élément acousto-mécanique réglable (201) comprend une pluralité respective de deuxièmes portions (205) configurées pour fermer au moins partiellement la pluralité d'ouvertures (209).

9. Dispositif de protection auditive (20) pour insertion dans un conduit auditif d'un sujet mammifère, comprenant un premier élément acousto-mécanique (1) réglable, dans lequel le premier élément acousto-mécanique (1) est un élément acousto-mécanique (1) réglable selon une quelconque revendication précédente.

10. Dispositif de protection auditive (20) selon la revendication 9, comprenant une entrée utilisable par l'utilisateur pour régler le premier élément acousto-mécanique (1) réglable.

11. Dispositif de protection auditive (20) selon la revendication 9 ou la revendication 10, comprenant un dispositif de commande (28) agencé pour commander l'agencement de réglage de manière à modifier la réponse acoustique de l'au moins un trajet sonore.

12. Dispositif de protection auditive (20) selon l'une quelconque des revendications 9 à 11, comprenant une entrée utilisable par l'utilisateur, qui permet à l'utilisateur de sélectionner le fonctionnement du dispositif (20).

13. Dispositif de protection auditive (20) selon l'une quelconque des revendications 9 à 12, dans lequel le premier élément acousto-mécanique (1) comprend une partie d'un ensemble d'éléments acoustiques, commandant la réponse en fréquence de l'atténuation.

14. Dispositif de protection auditive (20) selon l'une quelconque des revendications 9 à 13, comprenant en outre un deuxième élément acousto-mécanique (22) réglable, agencé acoustiquement en série avec le premier élément acousto-mécanique (1) réglable, le deuxième élément acousto-mécanique (22) réglable comprenant une membrane réglable.

15. Dispositif de protection auditive (20) selon la revendication 14, comprenant un agencement de réglage pour régler simultanément les premier et deuxième éléments acousto-mécaniques (1, 22) réglables pour modifier une réponse acoustique de l'au moins un trajet sonore, facultativement dans lequel l'agencement de réglage comprend un deuxième actionneur (26) pour régler le deuxième élément acousto-mécanique (22) réglable.
